# EUROPEAN PATENT APPLICATION

(11) **EP 4 772 638 A1**
(43) Date of publication of application: **08.07.2026**
(21) Application number: 25150023.7
(22) Date of filing: 02.01.2025
(51) Int. Cl.: C12N 15/113, A61K 31/7088

(54) **FUNCTIONAL OLIGONUCLEOTIDES FOR ENHANCED TRANSLATION OF SPECIFIC PROTEINS THROUGH BRIDGING RNAS**

(71) Applicant: Kemijski Institut, 1000 Ljubljana (SI)
(72) Inventor: JERALA, Roman, 1000 Ljubljana (SI); FORSTNERIC, Vida, 1000 Ljubljana (SI)
(74) Representative: Weickmann & Weickmann PartmbB

(57) **Abstract**

The present invention provides a functional nucleic acid for enhancing protein translation from a target mRNA in a cell. It further provides DNA molecules and expression vectors encoding the functional nucleic acids, as well as compositions, methods and uses related to the functional nucleic acids. The functional nucleic acids are useful for genetic research, protein production, and may particularly be used for the treatment of diseases associated with reduced gene expression, such as those caused by haploinsufficiency and other genetic or non-genetic disorders for which an increase in target protein level could be beneficial.

## Description

The present invention provides functional nucleic acids for enhancing specific protein translation from a target mRNA in a cell. It further provides DNA molecules and expression vectors encoding the functional nucleic acids, as well as compositions, methods and uses related to the functional nucleic acids. The functional nucleic acids are useful for genetic research, protein production, and may particularly be used for the treatment of diseases associated with reduced gene expression, such as those caused by haploinsufficiency and other genetic or non-genetic disorders for which an increase in a target protein level could be beneficial for therapy or biotechnological applications.

The present invention generally relates to nucleic acid molecules capable of enhancing protein translation, and more specifically to functional nucleic acids that enhance the translation of a selected target mRNA sequence through acting as a bridge between mRNAs.

Rare diseases (RDs) encompass a diverse group of disorders that collectively affect approximately 6% of the human population. Estimates suggest that a large majority, approximately 80%, of RDs have a known or presumed genetic etiology. Genetic RDs can result from various genetic alterations, including the involvement of one or several genes or chromosomal abnormalities. For many genes, the deletion or mutation of one functional copy may result in the impaired activity of the protein, leading to an abnormal or disease state. These genes are termed haploinsufficient as one copy is insufficient to produce a normal or wild-type phenotype. Large-scale exome sequencing analyses estimate that there could be over 3000 human genes that are haploinsufficient (Lek et al., Nature 536, 285 (2016)). Many of the observed phenotypes resulting from the loss of function or deletion of a single copy of a haploinsufficient gene are associated with conditions such as intellectual disability, developmental or metabolic disorders, and tumorigenesis. Upregulation of specific proteins in a cell could also be beneficial even if this specific protein is not disrupted by the disease.

Recent years have seen fast developments in the field of functional nucleic acids and their use as therapeutics. Such therapeutics also hold great potential in the treatment of genetic diseases, including genetic rare diseases and conditions associated with haploinsufficiency.

Developments in synthetic nucleotide chemistry have improved stability and immunogenicity of nucleotides and are widely applicable to different classes of RNA-targeted therapeutics. Oligonucleotides are typically large, hydrophilic polyanions and do not readily pass through the plasma membrane. Furthermore, they may be sequestered by certain plasma proteins or removed by phagocytic immune cells. Chemical modification significantly enhances oligonucleotide drug stability and delivery by improving pharmacodynamics, pharmacokinetics, and biodistribution through modifications to the nucleic acid backbone, ribose sugar, and nucleobases. Notably, extensive chemical modification alone enables tissue delivery of antisense oligonucleotides (ASOs), evident in several approved oligonucleotide therapies (Hung et al., Nucleic Acid Ther. 23, 369-378 (2013)). Naked ASOs have been developed for diseases such as amyotrophic lateral sclerosis, Alzheimer's disease, centronuclear myopathy, and Huntington's disease, relying solely on chemical modification for tissue delivery of the oligonucleotides in a naked form. Administering chemically modified oligonucleotides directly into the cerebrospinal fluid through lumbar puncture has shown to achieve a favorable distribution of therapeutic molecules, such as nusinersen, across the central nervous system (Tabrizi et al., N. Engl. J. Med. 380, 2307-2316 (2019)). Common chemical modifications include the incorporation of phosphorothioate (PS) linkages in the backbone which confers nuclease resistance and may promote binding to certain plasma and cellular proteins, improving drug pharmacokinetics by increasing the circulation time. Other modifications of currently approved oligonucleotide therapeutics include modifications or substitutions of 2'-OH in ribose, such as 2'-O-methyl, 2'fluoro and 2'-O-(2-methoxyethyl) which also enhance endonuclease resistance and serum stability. Modifications such as locked nucleic acids additionally increase stability of RNA-target duplexes. Another strategy is delivery of oligonucleotides packaged into lipid nanoparticles (LNPs) or in complex with lipids or peptides or their mimetics. LNP-based delivery of in vitro transcribed (IVT) mRNA is well established and was successfully applied for delivery of RNA vaccines (Egli et al., Nucleic Acids Res. 51, 2529-2573 (2023)).

Nucleic acid-based therapies (NBTs) have already seen success in the clinic due in part to their transient effect and ability to regulate the dose and therefore higher safety profile compared to gene editing or gene replacement therapy. To date, targeting approaches have predominantly focused on gene silencing, e.g. siRNAs and antisense oligonucleotides (ASOs), as gene silencing with nucleic acids is generally easier to achieve. However, in many conditions including those related to haploinsufficiency of a gene, the opposite effect is desired, i.e., increasing expression of a specific gene. Thus, an urgent need remains for strategies and therapeutic compounds that are able to increase the level of a protein of interest.

Strategies for increasing protein levels may either increase mRNA abundance or optimize translation.

Strategies from the first group include therapeutic mRNA delivery, transcript stabilizers, inhibition of naturally occurring antisense transcripts (NATs) or activators and splicing modulators that prevent nonsense-mediated decay (NMD). Antisense oligonucleotides (ASOs) binding to specific pre-mRNA sequences regulating splicing can impede the generation of mutated or non-productive transcripts, thereby boosting target protein levels. Significant clinical success has been noted for several therapeutic splice modifying oligonucleotides, such as Nusinersen, approved to treat spinal muscular atrophy (SMA), eteplirsen for treatment of Duchenne muscular dystrophy (DMD) and sepofarsen for treatment of Leber congenital amaurousis (Khorkova et al., Nat. Rev. Drug Discov. 22, 539-561 (2023)). Another approach is the so-called targeted augmentation of nuclear gene output (TANGO) platform. TANGO is based on the discovery of naturally occurring 'toxic' exons present in some mRNAs, which are destroyed through the NMD pathway. Rather than targeting a specific mutation, mRNA levels are upregulated through prevention of inclusion of toxic exons. An example is a splice-switching oligonucleotide targeting SCN1 for treatment of Dravet syndrome which is currently in clinical trials (Khorkova et al., Nat. Rev. Drug Discov. 22, 539-561 (2023)). Recently discovered small activating RNAs (saRNAs) for gene upregulation contain a guide strand complementary to a promotor or enhancer region and show promising results in phase I clinical trials in cancer supplemental therapy. However, the detailed mechanism of action is still not completely clear and it has been difficult to identify saRNAs for any desired target (Hashimoto et al., Clin. Cancer Res. Off. J. Am. Assoc. Cancer Res. 27, 5961-5978 (2021)).

Strategies from the second group, for optimizing translation, may rely on disengaging endogenous translation inhibitory control mechanisms such as upstream open reading frame (uORF) translation or inhibitory secondary structures in the 5'UTR region. Alternatively, they may utilize non-coding RNAs (ncRNA) that regulate translation initiation and elongation. The two former strategies are limited to genes which, first, contain such regulatory elements and second, for which these regulation mechanisms have been studied in detail. The latter approach, use of long non-coding RNA for recruitment of eukaryotic translation machinery to the mRNA of interest, has shown some promising results in vitro and in vivo (Espinoza et al., Essays Biochem. 65, 775-789 (2021)). However, the length of these molecules (250-1200 nt) exceeds the maximal lengths feasible for chemical synthesis and delivery by gymnosis, limiting stability and administration options. A similar approach utilized viral or endogenous IRES sequences fused to a modular guide RNA (gRNA) sequence which increased protein levels through recruitment of cellular translational machinery to a target mRNA in vitro and in vivo(Cao et al., Nat. Commun. 14, (2023)). However, these RNA molecules showed short-lived effects and also exceed current size limitations of efficient synthesis of chemically modified oligonucleotides of high yield and purity.

Therefore, there persists an unmet need for scalable, modular technology that could be adapted for upregulation of a diverse specific proteins of interest for tissue or cell types where insufficiency of these proteins of interest poses a problem and may lead to pathogenic phenotypes or where cells could be modified into a different state. Ideally, these molecules should be able to synthesize by chemical synthesis and exhibit high biological stability and good pharmacokinetic qualities for use in vivo or ex vivo.

The present invention surprisingly solves this task by making use of the high translational activity of known genes such as housekeeping genes. Using a small nucleic acid molecule, mRNAs of such highly translated genes are recruited to any target mRNA of interest to boost its translational activity. Surprisingly, introduction of such nucleic acid molecules into cells was shown to increase target protein translation by 1.5 to 3 fold. Such an increase is sufficient to provide a therapeutic effect in many conditions, including haploinsufficiency of many genes or to modify the state of cells in differentiation. The small size of the functional nucleic acids described in this invention allows the use of nuclease resistant chemically synthesized molecules which can easily be delivered to the target cells or tissues and provide long lasting effect.

Thus, in a first aspect, the present invention provides a functional nucleic acid comprising:
(A) a target binding sequence essentially reverse complementary to a region of a target mRNA sequence for which protein translation in a cell is to be enhanced, and
(B) a donor binding sequence essentially reverse complementary to a region of a donor mRNA sequence which has a high translational activity in said cell.

Without wishing to be bound by a theory, it is assumed that the functional nucleic acid tethers the donor and target mRNA at least transiently in the cell. As the donor mRNA having high translational activity is highly efficient at recruiting initiation factors and ribosomes, it is assumed that recruiting the target mRNA into such an environment may efficiently boost its translation initiation rate, and thereby enhance its translation. Starting from this inventive concept, the inventors have shown experimentally that it works in practice for various donor and target mRNAs. Thus, the present invention provides an easy and robust new approach to enhancing translation of any target mRNA in a cell. The approach is highly modular, as any donor mRNA may be combined with any target mRNA or even multiple mRNAs. This allows easy tuning of the approach, such as for optimizing translation enhancement for specific cells, conditions and target genes. As a further advantage, the functional nucleic acid may be very short and may thus be synthesized chemically as a modified nucleic acid to improve its pharmacodynamics, pharmacokinetics, and biodistribution. Further the donor mRNA may be characteristic for a certain cell type or cell state and may therefore stimulate upregulation of a selected target protein in a cell- or tissue-specific manner.

The functional nucleic acid may be designed to function in translation enhancement in any eukaryotic or prokaryotic cell, preferably a eukaryotic cell. The functional nucleic acid may be functional in an isolated cell and/or in a cell in a tissue or organism. Preferably, the sequence of the functional nucleic acid is designed based on the inventive concept and the preferred features described herein, using publicly available data and simple computational tools known to the person skilled in the art. Given the robustness of the inventive approach as demonstrated herein, a skilled person may design a functional nucleic acid according to the invention without performing any experiments.

Preferably, the functional nucleic acid of the invention is an RNA molecule. In one preferred embodiment, the functional nucleic acid is a nonmodified or native RNA molecule, i.e., an RNA molecule without any chemical modifications that can be delivered as such to cells or an RNA that is transcribed from DNA delivered into cells in a form of a plasmid, viral or other vector. In an alternative preferred embodiment, the functional nucleic acid is a chemically modified RNA molecule comprising at least one chemical modification compared to a native RNA molecule. The terms "native" and "nonmodified" may be used interchangeably herein.

The inventors have shown experimentally that both nonmodified and chemically modified functional nucleic acids, particularly RNA molecules, can effectively enhance translation of target mRNAs in cells (see Examples 2-9, particularly Example 9 using a fully chemically modified ASO).

The functional nucleic acid of the invention is preferably a single-stranded nucleic acid molecule. It is preferred that the functional nucleic acid does not form stable intramolecular secondary structures under physiological conditions. This ensures that the functional nucleic acid can efficiently bind to both its complementary regions in the target and donor mRNA sequences. Tools and programs to predict formation and stability of secondary structures of nucleic acids are well known in the art. For example, it is preferred that the functional nucleic acid has a sequence which cannot hybridize intramolecularly over a length of 5 consecutive base pairs or more. Physiological conditions are known in the art to be conditions in a temperature, pH and osmolarity range suitable for living cells, preferably for cells in which protein translation from the target mRNA is to be enhanced.

Thus, in a particularly preferred embodiment, the functional nucleic acid of the invention is a single-stranded RNA molecule which does not form stable secondary structures under physiological conditions and which is preferably a native or a chemically modified RNA molecule.

The functional nucleic acid may be an isolated nucleic acid molecule, preferably produced by chemical synthesis. Alternatively, the functional nucleic acid may be produced *in situ,* i.e., in a target cell, through transcription from a DNA or expression vector.

If the functional nucleic acid of the invention is a chemically modified RNA molecule, it is preferably produced by chemical synthesis.

In a preferred embodiment, at least one internucleosidic linkage in the functional nucleic acid of the invention is a modified internucleosidic linkage. The modified internucleosidic linkage is preferably a modified nuclease resistant internucleosidic linkage, more preferably a phosphorothioate linkage.

A phosphorothioate linkage is a chemical modification in the backbone of nucleic acids where a non-bridging oxygen atom is replaced by sulfur, enhancing stability against nucleases.

In a further preferred embodiment, at least two internucleosidic linkages, even more preferably 20-100%, in particular 40-100%, of the internucleosidic linkages in the functional nucleic acid of the invention are modified internucleosidic linkages as defined above.

In a particularly preferred embodiment, all internucleosidic linkages in the functional nucleic acid are modified internucleosidic linkages, preferably phosphorothioate linkages.

In a preferred embodiment, at least one nucleotide in the functional nucleic acid is a 2'-modified ribonucleotide, preferably selected from the group consisting of 2'-O-methylribonucleotide, 2'fluororibonucleotide and 2'-O-(2-methoxyethyl)-ribonucleotide (2'MOE).

Such 2' modifications of ribonucleotides have been shown to increase their stability, binding affinity, and resistance to enzymatic degradation.

In a particularly preferred embodiment, all nucleotides in the functional nucleic acid are 2'-modified ribonucleotides, preferably 2'MOE-ribonucleotides.

In a further preferred embodiment, at least one nucleotide in the functional nucleic acid is a 2'-4'-bridged nucleotide, preferably a locked nucleotide having a 2'-O-4'-C-methylene bridge.

In a particularly preferred embodiment, the functional nucleic acid of the invention is a chemically modified RNA molecule comprising one or more chemical modifications selected from a phosphorothioate internucleosidic linkage, a 2'-modified ribonucleotide, preferably selected from a 2'-O-methylribonucleotide, 2'fluororibonucleotide or 2'-O-(2-methoxyethyl)-ribonucleotide, or a locked nucleotide.

In a further particularly preferred embodiment, the functional nucleic acid of the invention is a chemically modified RNA molecule wherein all internucleosidic linkages are phosphorothioate (PS) linkages, and all nucleotides have a 2'MOE-modification.

In the functional nucleic acid of the invention, the target binding sequence (A) may be upstream or downstream from the translated region, i.e., located at the 5' region or at the 3' region of the donor binding mRNA (B), preferably at the 5' region. The inventors have shown experimentally that both configurations may enhance translation of the target mRNA with similar efficiency (see Example 7 below).

Preferably, the functional nucleic acid of the invention has a total length of 20 to 100 nucleotides, more preferably of 25 to 50 nucleotides, where none of the donor or acceptor binding domain should preferably be longer than 20 nucleotides.

Thus, the functional nucleic acid of the invention preferably lies within a size range for which chemical synthesis is feasible. Oligonucleotides having up to around 60 nucleotides can generally be synthesized routinely using methods known to one skilled in the art. Chemical synthesis of the functional nucleic acid of the invention allows incorporation of appropriate chemical modifications as discussed above, which improve pharmacokinetic and pharmacodynamics properties facilitating delivery, durability and decreased immunoreactivity *in vivo.* The small size thus constitutes a major advantage compared to other translation-activating nucleic acid molecules known in the art.

In a preferred embodiment of the invention, the functional nucleic acid is covalently or non-covalently linked to another biopolymer, preferably to another nucleic acid or a protein or a lipid.

### [Target mRNA/ target binding sequence]

The target binding sequence (A) of the functional nucleic acid of the invention is essentially reverse complementary to a region of a target mRNA sequence for which protein translation in a cell is to be enhanced. The target mRNA may also be called acceptor or acceptor mRNA.

The target mRNA may be any mRNA for which protein translation in a cell is to be enhanced. This implies that the target mRNA must be present in the cell. It is preferred that the target mRNA is present in the cell in an accessible form for translation, preferably in the cytosol. It is further preferred that the target mRNA is present in a sufficient copy number for binding between the target mRNA and the functional nucleic acid to occur in the cell or it may be induced by an external signal known in the art to induce the transcription of the target mRNA in a cell. Preferably, the target mRNA is an endogenous transcript of an endogenous gene in the cell.

In a preferred embodiment, the target mRNA may be a transcript of a haploinsufficient gene in the cell. In this case, the functional nucleic acid may be used to enhance translation of the transcript of the haploinsufficient gene, thereby alleviating the effects of the missing or mutated allele. However, apart from presence in the cytosol of the cell, the target mRNA is not particularly limited. For instance, the target mRNA may also be an mRNA present at physiological levels in the cell, whose translation is to be enhanced for other reasons.

In another preferred embodiment the target mRNA may be a transcript that codes for a protein that can cause some desired beneficial effect in the cells. In this case, the functional nucleic acid may be used to enhance translation of the transcript of the target gene, thereby triggering some beneficial action in the cell. For instance, the target mRNA may also be an mRNA present at physiological levels in the cell, and the increased level of this protein as a result of the functional nucleic acid may differentiate the cell to a beneficial state or prevent some process within a cell.

The embodiment of functional nucleic acid is not limited to a single functional acid as several different functional nucleic acids could be used at the same time in the cell which result in upregulation of several different proteins, which may collectively have a beneficial effect. Multiple functional nucleic acids may have different acceptor and donor domains or some domains may use the same donor domain or the same acceptor domain. The number of multiple functional domains is not limited but is preferably in the range between 2 and 20 functional nucleic acids.

The minimal number of binding domains within the functional nucleic acids is two, comprising one donor and one acceptor domain, however the number of binding domains within the functional nucleic acid is not limited to two and can contain multiple acceptor or multiple donor domains or both. Multiple domains can enhance the upregulation of a target protein or enhance upregulation of multiple proteins. The preferred number of multiple domains is limited by the ability to synthesize functional nucleic acids by chemical synthesis and their ability to be delivered to cells and by the formation of secondary structure within the functional nucleic acid. The preferred number of binding or acceptor domains is in the range from 1 to 5 domains, more preferably 1 and 2 domains.

The inventors have shown experimentally that translation of different mRNAs with highly different 5'UTRs and predicted translation efficiencies can be boosted efficiently with functional nucleic acids according to the invention (see Examples 1-3 and 9). This shows that the inventive concept is generally applicable and not limited to any particular target mRNA.

The target binding sequence (A) in the functional nucleic acid of the invention preferably has a length of 8 to 50 nucleotides, more preferably of 14 to 22 nucleotides.

As known by one skilled in the art, base complementarity describes the specific pairing of nucleobases in DNA or RNA through hydrogen bonding: adenine (A) with thymine (T) or uracil (U), and cytosine (C) with guanine (G). Thus, a fully reverse complementary sequence to a first sequence comprises the complementary bases to the first sequence in reverse order, allowing it to pair with the first sequence with both sequences oriented in opposite directions (i.e., the 5'-end of the first sequence pairs with the 3' end of its fully reverse complementary sequence). For sequences, the terms "complementary" and "reverse complementary" may be used interchangeably herein, as it is commonly done in the art.

As used herein, "essentially reverse complementary" means that the respective sequences are 75-100%, preferably 80-100%, reverse complementary to each other. Essentially reverse complementary sequences may also be fully complementary. Importantly, sequences which are essentially but not fully reverse complementary can still bind to each other through base pairing. The melting temperature of the bound duplex may however be reduced compared to a duplex formed from the respective fully reverse complementary sequences.

If a sequence is less than 100% complementary to the other sequence, it consists of complementary and non-complementary segments. Non-complementary segments in the sequences may comprise nucleobase exchanges, insertions or deletion. Preferably, for any sequence in the functional nucleic acid of the invention that is essentially reverse complementary to another sequence, any non-complementary segment that may be present is small, with a preferred length of 1 to 5 nucleotides, preferably 1-3 nucleotides, at the most. Nucleobase exchanges are preferred over insertions or deletions. It is further preferred that each fully complementary segment in the sequence has a minimum length of 6 nucleotides.

Thus, in a preferred embodiment, the target binding sequence (A) is at least 80% complementary to a region of the target mRNA sequence. It is further preferred that the target binding sequence comprises one or more segments fully complementary to a region of the target mRNA sequence, wherein each fully complementary segment is at least 6 nucleotides long.

In a particularly preferred embodiment, the target binding sequence is fully reverse complementary to a region of the target mRNA sequence.

Preferably, the target binding sequence (A) in the functional nucleic acid of the invention is essentially reverse complementary to a region of the target mRNA sequence which does not form and is not part of a stable intramolecular secondary structure under physiological conditions. The region of the target mRNA that the target binding sequence (A) is essentially reverse complementary to may also be called the target sequence or target motif.

The target binding sequence (A) is preferably essentially reverse complementary to a region comprised in the 5' untranslated region (5'UTR), the coding sequence (CDS) and/or the 3' untranslated region (3'UTR) of the target mRNA. Thus, the target sequence may be fully comprised in the 5'UTR, in the CDS or in the 3'UTR of the target mRNA, or it may be at an intersection between these functional regions and comprised in the 5'UTR and the CDS, or in the CDS and the 3'UTR. Preferably, the target sequence is either comprised in the 5'UTR or in the 3'UTR of the target mRNA.

The term "5' untranslated region (5' UTR)", as used herein, refers to the non-coding sequence at the beginning of an mRNA transcript upstream of the coding region. It plays roles in the regulation of translation efficiency and mRNA stability.

The term "3' untranslated region (3' UTR)", as used herein, refers to the non-coding sequence at the end of an mRNA transcript downstream of the coding region. It contains regulatory elements that influence mRNA stability, localization, and translation.

The term "coding sequence (CDS)", as used herein, refers to the portion of a gene or mRNA that encodes the amino acid sequence of a protein. This sequence is translated by ribosomes during protein synthesis.

In one preferred embodiment, the target binding sequence (A) is essentially reverse complementary and preferably fully reverse complementary to a region comprised in the 5' untranslated region (5'UTR) of the target mRNA which does not form and is not part of a stable intramolecular secondary structure under physiological conditions.

In one preferred embodiment, the target binding sequence (A) is essentially reverse complementary and preferably fully reverse complementary to a region comprised in the 3' untranslated region (3'UTR) of the target mRNA which does not form and is not part of a stable intramolecular secondary structure under physiological conditions.

### [Donor binding sequence / donor mRNA ]

The donor binding sequence (B) of the functional nucleic acid of the invention is essentially reverse complementary to a region of a donor mRNA sequence which has a high translational activity in the cell. The region of the donor mRNA that the donor binding sequence (B) is essentially reverse complementary to may also be called the donor sequence or donor motif.

Preferably, the donor binding sequence has a length of 6-50 nucleotides, preferably 6-22 nucleotides, more preferably of 6-20 nucleotides.

In a preferred embodiment, the donor binding sequence (B) is at least 80% complementary to a region of the donor mRNA sequence. It is further preferred that the donor binding sequence comprises one or more segments fully complementary to a region of the donor mRNA sequence, wherein each segment is at least 6 nucleotides long.

In a particularly preferred embodiment, the donor binding sequence is fully reverse complementary to a region of the donor mRNA sequence.

It is preferred that the donor-binding sequence is essentially reverse complementary to a region of the donor mRNA sequence which does not form and is not part of a stable intramolecular secondary structure under physiological conditions.

The donor binding sequence (B) is preferably essentially reverse complementary to a region comprised in the 5' untranslated region (5'UTR), the coding sequence (CDS) and/or the 3' untranslated region (3'UTR) of the donor mRNA. Thus, the donor sequence may be fully comprised in the 5'UTR, in the CDS or in the 3'UTR of the donor mRNA, or it may be at an intersection between these functional regions and comprised in the 5'UTR and the CDS, or in the CDS and the 3'UTR. Preferably, the donor sequence is either comprised in the 5'UTR or in the 3'UTR of the donor mRNA.

In one preferred embodiment, the donor binding sequence (B) is essentially reverse complementary and preferably fully reverse complementary to a region comprised in the 5' untranslated region (5'UTR) of the donor mRNA which does not form and is not part of a stable intramolecular secondary structure under physiological conditions.

In one preferred embodiment, the donor binding sequence (A) is essentially reverse complementary and preferably fully reverse complementary to a region comprised in the 3' untranslated region (3'UTR) of the donor mRNA which does not form and is not part of a stable intramolecular secondary structure under physiological conditions.

The donor mRNA is an mRNA that has a high translational efficiency in the cell. As used herein, "high translational efficiency" means that translation of the mRNA leads to a high protein level/number derived this mRNA which is above the average in comparison ratio relative to other mRNAs transcripts in the cell. As the rate of translation initiation is usually limiting for translation, the ribosome density on an mRNA is generally a good and well-accepted measure of its translational activity.

Various methods are available to one skilled in the art to estimate translational activity of an mRNA through experiments or through the data available in scientific literature. The ribosome density on an mRNA may for example be estimated by a method called polysome profiling. Here, the different ribosomal species in a cell extract - small and large subunits, single ribosomes and polysomes - are separated in a sucrose gradient. In each fraction, the amount of target mRNA is determined, using common methods such as Northern blot, RT-qPCR, cDNA microarrays or RNA-seq. A strong association of an mRNA with ribosomes and especially with polysomes indicates high translational activity. The analysis of the ratio of protein to mRNA from databases indicates high translational activity.

More recently, reliable tools have also been developed to predict the translational activity of mRNAs with good confidence. As mentioned above, the rate-limiting step of translation is initiation wherein the ribosome complex is recruited to the mRNA initiation codon by a multifaceted network of eukaryotic initiation factors (elFs). Transcripts with strong 5' UTR regions direct high levels of ribosome loading which results in high levels of protein production. A recently developed algorithm based on polysome profiling and deep learning allows prediction of 5' UTR strength for any gene of interest and describes this strength in relative units of mean ribosomal load (MRL) on a scale of 0 to 10 (Sample et al., Nat. Biotechnol. 37, 803-809 (2019)).

The term "mean ribosomal load (MRL)", as used herein, describes with relative scoring the average number of ribosomes bound to a single mRNA molecule during translation, reflecting translational efficiency.

It is preferred that the donor mRNA has a translational activity equal to or higher than the 50th percentile of the distribution of translational activities of the mRNA population in the cell. Thus, it is preferred that the donor mRNA has a predicted mean ribosomal load equal to or higher than the 50th percentile of MRL of mRNAs in the cell. It is particularly preferred that the donor mRNA is among the 100 mRNAs with the highest predicted MRL in the cell. If MRL is indicated on a scale from 0 to 10, with 10 being the highest MRL observed for an mRNA in the cell, the MRL of the donor mRNA is preferably ≥ 5. The MRL is preferably predicted using a deep learning model trained and validated on experimental data on translational activity, such as on polysome profiling data. As a preferred example, the MRL may be predicted using the "Optimus 5-Prime" model provided by Sample and colleagues. However, other algorithms may be used as well to predict MRLs of mRNAs. In another preferred embodiment, the MRL may be derived directly from experimental data.

Preferably, the donor mRNA has a higher translational activity than the target mRNA in the cell. More preferably, the translational activity of the donor mRNA is at least 20% higher than the translational activity of the target mRNA. The relative translational activities of donor and target mRNA may be assessed by predicted MRL. Thus, preferably, the ratio of predicted mean ribosome load (MRL) of the donor mRNA to MRL of the target mRNA is 1.2:1 to 3:1.

It is further preferred that the donor mRNA is highly abundant in the cell. Preferably, the donor mRNA is more abundant in the cell than the target mRNA. It is preferred that the ratio of (estimated) copy numbers of donor and target mRNA transcripts present in the cell is from 5:1 to 100:1, more preferably 1.5:1 to 100:1. mRNA abundance in a cell may for example be assessed with quantitative reverse transcription polymerase chain reaction (RT-qPCR). Alternatively, the mRNA abundance may be estimated from published data, such as from a publicly available database. For example, mRNA levels of human genes in various tissues, cell types and cell lines are available in the Human Protein Atlas (https://www.proteinatlas.org/).

In a preferred embodiment, the donor mRNA is a transcript of a constitutively expressed gene, also called a housekeeping gene. The term "gene expression", as used herein, describes protein production from a gene, i.e., the whole process involving both transcription and translation. A highly expressed gene usually comprises high transcriptional activity and high translational activity. The term "housekeeping gene," as used herein, describes a gene that is ubiquitously expressed at stable levels in all cells, providing essential functions for basic cellular maintenance and survival. Housekeeping genes are well known to the person skilled in the art.

In another preferred embodiment, the donor mRNA is a transcript of a gene which is highly expressed within a specific cell type or tissue. As used herein, a cell type may be a cell type occurring in a healthy tissue and/or organism, or it may be a diseased cell type, such a cancer cell type. Preferably, said gene is not expressed or expressed at low levels in cells other than those of the specific cell type or tissue. Such genes are known in the art and/or may be found experimentally or in a database. A cell type- or tissue-specific donor mRNA may be used advantageously to specifically enhance protein translation from the target mRNA sequence in the cells of the specific cell type or tissue. This may reduce side effects of the functional nucleic acid of the invention when administered to an organism.

In another preferred embodiment, the donor mRNA is a transcript of a gene which is highly expressed under certain physiological conditions or in a specific cell state or characteristic for a certain cell type. Examples for such a condition or cell state include inflammation, starvation, oxidative stress, osmotic stress, hypoxia, heat shock, etc. Again, such a donor mRNA may be used to enhance specificity and reduce side effects of the functional nucleic acid of the invention, e.g. when administered to an organism.

The above criteria may also be combined advantageously. Thus, in a preferred embodiment, the donor mRNA is a transcript of a gene which is highly expressed in a specific cell type or tissue under certain physiological conditions or in a specific cell state. Again, such a donor mRNA may be used to enhance specificity and reduce side effects of the functional nucleic acid of the invention, e.g. when administered to an organism.

It is particularly preferred that the donor mRNA is a transcript of a housekeeping gene. In a preferred embodiment, the donor mRNA encodes a protein selected from the group consisting of TUBA1A, SNRPD3, RPSX4, RPSX4, EEF1G, ACTB, HPRT, TPT1, YWHAZ, VPS29, RPLPO, RPLPO, B2M, SDHA, PSMB2, CHMP2A,ACTG1, RPL13A, RPL12, EEF1A1, C1orf43, GAPDH, HBB, UBB, UBC, UBA52 and PGK1.

The inventors have shown that the transcripts of various housekeeping genes with different cellular functions - TUBA1A, SNRPD3, RPSX4 - may all be used as donor mRNA according to the present invention (see e.g. Examples 2-4 and 8). This shows that the sequence or identity of the donor mRNA is not particularly limited, but that any donor mRNA may be used as long as it meets the criterium of high translational activity.

In a preferred embodiment of the invention, the functional nucleic acid further comprises a linker sequence between the target binding sequence (A) and the donor binding sequence (B). The presence of a linker sequence facilitates simultaneous binding of the functional nucleic acid to its complementary regions in the target and donor mRNA. While the presence of a linker is preferred, to facilitate base pairing along the full complementary region, it is not essential to the invention. The term "linker," as used herein, describes a short molecular sequence or structure used to connect functional motifs or domains, enabling proper spatial orientation or interaction.

The linker sequence is preferably not complementary to the target mRNA or the donor mRNA. In particular, the linker sequence is preferably not complementary to regions adjacent to, i.e. directly upstream or downstream of the target sequence and the donor sequence. Further, it is preferred that the linker sequence does not extend the complementarity together with the border regions of the acceptor of donor binding domains to any other mRNA in the cell over a length of 14 nucleotides or more.

If present, the linker sequence has preferably a length of 1 to 50 nucleotides, more preferably of 2 to 10 nucleotides. The inventors have shown experimentally that the length of the linker is not particularly limiting. In the experiment shown in Example 7 below, the different tested linker lengths (5, 7 and 9 nt) did not affect translation enhancement by the functional nucleic acid.

In a particularly preferred embodiment of the invention,
- the functional nucleic acid of the invention, which is preferably an RNA molecule, has a total length of 20 to 100 nucleotides, preferably 25 to 50 nucleotides, and
- the target binding sequence (A) has a length of 8 to 50 nucleotides, preferably 14 to 22 nucleotides, and
- the donor binding sequence (B) has a length of 6 to 50 nucleotides, preferably 6 to 22 nucleotides, and,
- if present, the linker sequence between (A) and (B) has a length of 1 to 50 nucleotides, preferably of 2 to 10 nucleotides.

In a preferred embodiment the functional nucleic acid consists of the target binding sequence (A) and the donor binding sequence (B), wherein the lengths of the functional nucleic acid, (A) and (B) are preferably as defined above.

In an alternative preferred embodiment, the functional nucleic acid consists of the target binding sequence (A), the linker sequence and the donor binding sequence (B), wherein the lengths of the functional nucleic acid, (A), (B) and the linker sequence are preferably as defined above.

### [DNA / expression vector]

In a further aspect, the present invention provides a DNA molecule encoding the functional nucleic acid of the invention.

In a further aspect, the present invention provides an expression vector comprising a sequence encoding the functional nucleic acid of the invention.

As will be understood by one skilled in the art, when produced by transcription of the DNA molecule or of the sequence comprised in the expression vector, the functional nucleic acid is preferably an RNA molecule, more preferably an RNA molecule transcribed in cells.

The expression vector may be for transient or stable production of the functional nucleic acid of the invention. Preferably, the expression vector is selected from a plasmid, linear or branched DNA or a viral vector, wherein the viral vector is preferably selected from a lentiviral, retroviral, adenoviral or AAV vector.

The term "plasmid," as used herein, refers to a circular, double-stranded DNA molecule capable of independent replication within a host cell, often used as a vector in genetic engineering.

The term "viral vector," as used herein, refers to a modified virus designed to deliver genetic material into target cells. Viral vectors are engineered to remove or disable their ability to cause disease while retaining their natural capacity to efficiently transfer nucleic acids. They are widely used in molecular biology research, gene therapy, and vaccine development. Viral vectors may be derived from various viruses, such as retroviruses, lentiviruses, adenoviruses, or adeno-associated viruses (AAV), depending on the application and desired properties. These vectors can facilitate stable or transient gene expression within the host cells, depending on the type of virus and the experimental or therapeutic context.

As is commonly known in the art, an expression vector may comprise further elements that enable its proper function, such as its replication and expression of the sequence encoding the functional nucleic acid of the invention. For example, the expression vector may comprise regulatory sequences operably linked to the sequence that is to be expressed, such as a promoter. Suitable expression vectors are known to one skilled in the art.

In another aspect, the present invention provides a composition comprising the functional nucleic acid, the DNA molecule or the expression vector as defined above. The composition may be a pharmaceutical preparation. In a preferred embodiment, the composition further comprises at least one pharmaceutically acceptable excipient. Preferably, the functional nucleic acid, the DNA molecule or the expression vector of the invention are stable in the composition for an extended period of time. Optionally, the composition may comprise at least one other active agent.

### [Method]

In a further aspect, the present invention provides an *in vitro* method of enhancing translation of a target mRNA sequence in an isolated cell, comprising the steps of:
(i) providing a functional nucleic acid, a DNA molecule or an expression vector of the present invention, and
(ii) delivering the functional nucleic acid, DNA or expression vector to the cell.

Preferably, the presence of the functional nucleic acid in the cell, either due to its direct delivery or due to expression from the DNA or expression vector or by the delivered RNA, results in enhanced translation of the target mRNA sequence in the cell. Preferably, transcriptional regulation of the target mRNA is not affected by the functional nucleic acid of the invention. Thus, it is preferred that target mRNA levels are not or not to a large degree affected by the method of the invention, more specifically that the expression of the protein from the donor mRNA is not decreased by more than 10% and does not affect the properties of the cell through its decrease.

In a preferred embodiment, the functional nucleic acid is provided in step (i) by chemical synthesis. In this case, the functional nucleic acid is preferably chemically modified molecule, more preferably a chemically modified RNA molecule as defined above.

The isolated cell in the *in vitro* method of the invention may be a eukaryotic cell or a prokaryotic cell. Preferably, the isolated cell is a eukaryotic cell, more preferably a mammalian cell, even more preferably an isolated human cell. In a preferred embodiment, the isolated cell is a cell of a cell line, e.g. a human-derived cell line.

Various known methods can be used to deliver the functional nucleic acid, DNA or expression vector to the cell in step (ii). The functional nucleic acid, which may preferably be a short chemically modified RNA molecule, may be delivered in a naked state without the use of a transfection reagent, a process which is also called gymnosis. Alternatively, the functional nucleic acid may be delivered via lipid nanoparticle (LNP) based delivery. The delivery of the DNA or expression vector into the cell may employ established scientific techniques, including transformation and transfection methods. These may involve chemical induction, electroporation, microinjection, lipofection, sonication, or viral-mediated delivery, among others. The introduced DNA may integrate transiently or stably. A transient integration refers to oligonucleotides or vectors that do not incorporate into the host genome. Stable integration occurs when the DNA becomes part of the host cell's genome. Stable incorporation of the described DNA may be verified using markers. Marker DNA sequences may confer resistance to antibiotics or chemicals and may either be included in the same DNA vector or provided on a separate vector.

### [Use]

In a further aspect, the present invention provides a use of a functional nucleic acid, a DNA or an expression vector of the invention for enhancing protein translation from a target mRNA sequence. Protein translation from the target mRNA sequence may be enhanced in any cell that the functional nucleic acid, DNA or expression vector is delivered to, preferably in an isolated cell.

In a preferred embodiment, the functional nucleic acid, DNA or expression vector may be used for enhancing protein translation from a target mRNA sequence, preferably in an isolated cell, for research and/or recombinant protein production.

### [Nucleic acid for use in a method of treatment]

The functional nucleic acid of the present invention may be used in a method of treating a disease or condition caused by reduced gene dosage or reduced gene expression. The disease or condition may be a genetic disorder, i.e., a disease or condition caused by abnormalities in the genome, including mutations, deletions, duplications, or chromosomal alterations. These disorders may affect gene expression or protein function, leading to a wide range of phenotypic consequences.

Preferably, the functional nucleic acid may be used in a method of treating a disease or condition caused by haploinsufficiency of a gene. In Haploinsufficiency, mutation in a coding or non-coding region of one allele results in a pathogenic phenotype due to insufficient protein levels. The remaining one functional copy of a gene is inadequate to maintain normal cellular or physiological function. However, given that functional mRNA of the gene is present in the cell, enhancing its translation may help to alleviate the condition.

Additionally, the functional nucleic acid may be used to treat or prevent diverse physiological or pathological conditions in which the increased amount of one or several proteins is beneficial. These physiological or pathological conditions or applications may relate to processes such as immunity, metabolism, differentiation, aging, growth, fitness, regeneration, infection, cancer, hormonal disorders, cardiovascular health, neurological and psychiatric disorders, digestion, sleep, cell reprogramming, cell differentiation and many others.

Thus, in a further aspect, the present invention provides a functional nucleic acid as defined above for use in a method of treating a disease or condition caused by reduced gene dosage or reduced gene expression.

In a preferred embodiment, the functional nucleic acid is for use in a method of treating a disease or condition caused by haploinsufficiency of a gene.

In a further aspect, the present invention provides a composition comprising the functional nucleic acid as defined above for use in a method of treating or preventing a disease or condition caused by reduced gene dosage or reduced gene expression. Preferably, the composition is a pharmaceutical composition or preparation.

In a further aspect, the present invention provides a composition comprising the functional nucleic acid as defined above for use in a method of treating or preventing a disease or condition which can be treated by increased amount of a selected protein or a group of selected proteins. Preferably, the composition is a pharmaceutical composition or preparation.

### Brief description of the drawings

The following drawings form part of the present specification and are included to further demonstrate certain aspects of the present invention. The invention may be better understood by reference to one or more of these drawings in combination with the detailed description of specific embodiments presented herein.

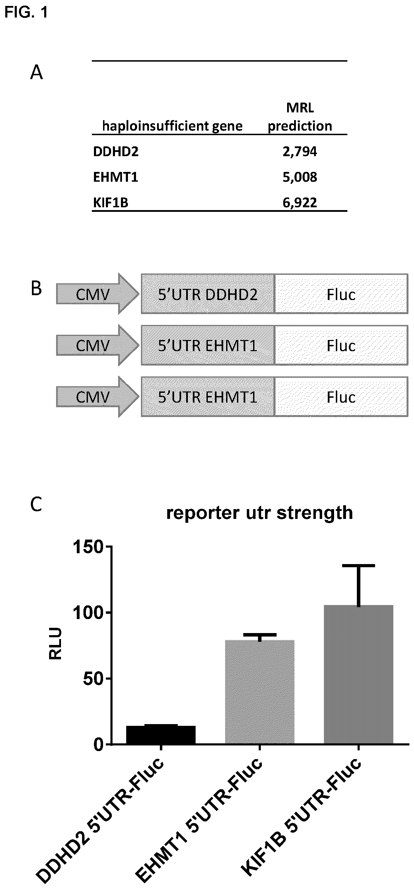

**FIG. 1****: (A)** Relative mean ribosomal load (MRL) levels on a scale of 1-10 predicted using the Optimus 5-Prime model (Sample et al., Nat. Biotechnol. 37, 803-809 (2019)) for haploinsufficient genes whose transcripts were selected as target mRNA (acceptor) according to the invention. **(B)** Schematic representation of reporter constructs prepared for testing functional nucleic acids according to the invention. Reporters comprise a sequence encoding the 5'UTR of the human variant of each selected gene cloned upstream of a sequence encoding firefly luciferase as a reporter. The sequences were cloned into the pCDNA3.1 expression vector for expression in mammalian cells. (C) HEK293 cells were transfected with 10 ng of the indicated reporter plasmid and luciferase signal was measured 24 h post transfection. Experimental results show correlation between predicted MRL levels and levels of luciferase expression.

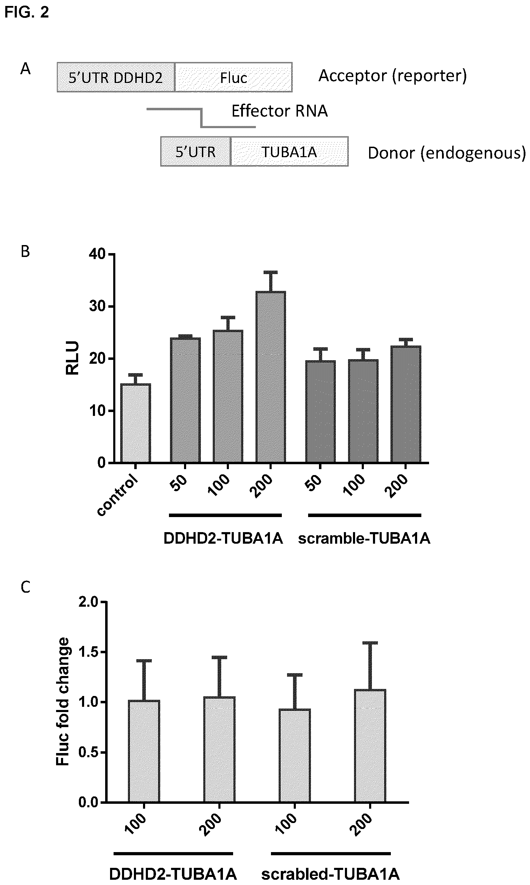

**FIG. 2****: (A)** Schematic representation of the mode of action of the functional nucleic acid of the invention. The functional nucleic acid is composed of a target (acceptor) binding sequence complementary to the 5'UTR of the target mRNA (acceptor), in this case the 5'UTR of the human DDHD2 gene. This motif is followed by a 5-nucleotide long linker and a sequence complementary to the donor 5' UTR, in this case the 5' UTR of the human TUBA1A gene. As target/reporter construct, the 5' UTR of DDHD2 was inserted upstream of a sequence encoding firefly luciferase (Fluc). Cells were transfected with a plasmid encoding the functional nucleic acid under control of the U6 promoter and with the target/reporter (acceptor) construct. The donor is endogenous. **(B,C)** HEK293 cells were transfected with the target/reporter construct only (control) or co-transfected with the target/reporter and 50, 100 or 200 ng/well of a plasmid encoding the functional nucleic acid (DDHD2-TUBA1A) or a control (scramble-TUBA1A) and **(B)** luminescence was measured after 24h. All samples were co-transfected with a plasmid encoding constitutively expressed renilla luciferase used for transfection normalization. (C) Using qPCR, fold change of *firefly luciferase* mRNA levels was analyzed with respect to a control sample transfected with the target/reporter only (control) and normalized to a house-keeping gene (GAPDH).

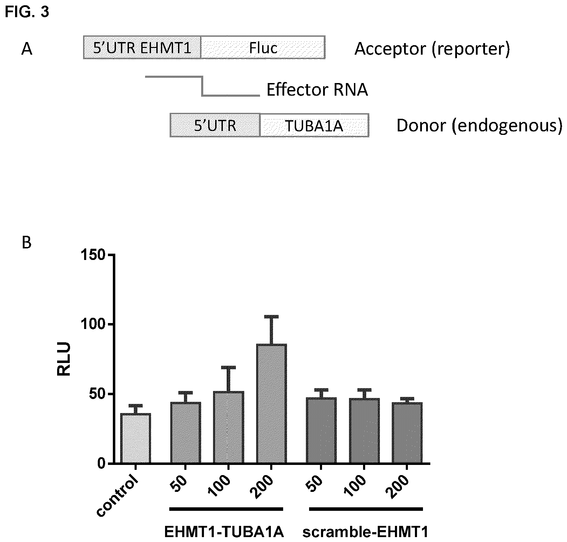

**FIG. 3****: (A)** Schematic representation of the mode of action of the functional nucleic acid. The functional nucleic acid is composed of a target (acceptor) binding sequence complementary to the 5'UTR of the target mRNA (acceptor), in this case the 5'UTR of the human EHMT1 gene. This motif is followed by a 5-nucleotide long linker and a sequence complementary to the donor 5' UTR, in this case the 5' UTR of the human TUBA1A gene. The 5' UTR of EHMT1 was inserted upstream of a sequence encoding firefly luciferase (Fluc) and is also referred to as the target/reporter construct. Cells were transfected with a plasmid encoding the functional nucleic acid under control of the U6 promoter and with the target/reporter (acceptor) construct. The donor is endogenous. **(B)** HEK293 cells were transfected with the target/reporter construct only (control) or co-transfected with the target/reporter and 50, 100 or 200 ng/well of a plasmid encoding the functional nucleic acid (EHMT1-TUBA1A) or a control (scramble-TUBA1A) and luminescence was measured after 24h. All samples were co-transfected with a plasmid encoding constitutively expressed renilla luciferase used for transfection normalization.

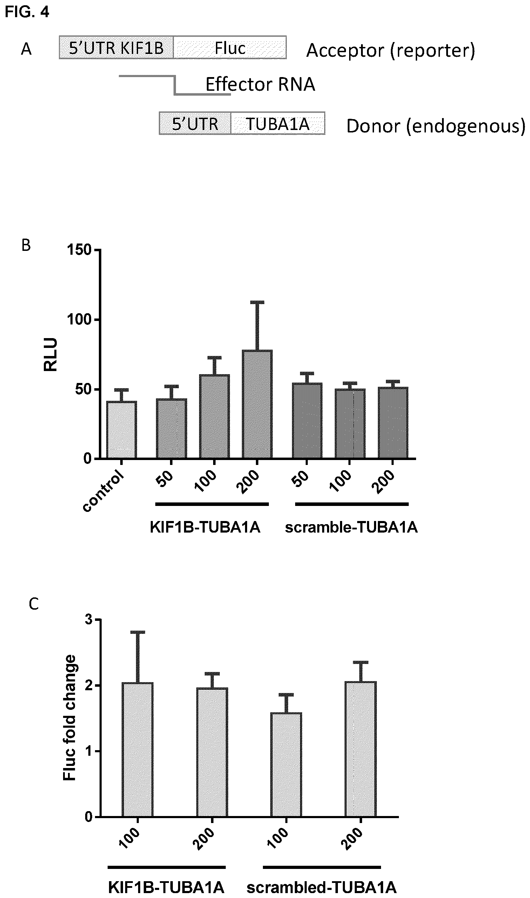

**FIG. 4****: (A)** Schematic representation of the mode of action of the functional nucleic acid. The functional nucleic acid is composed of a target (acceptor) binding sequence complementary to the 5'UTR of the target mRNA (acceptor), in this case the 5'UTR of the human KIF1B gene. This motif is followed by a 5-nucleotide long linker and a sequence complementary to the donor 5' UTR, in this case the 5' UTR of the human TUBA1A gene. The 5' UTR of KIF1B was inserted upstream of a sequence encoding firefly luciferase (Fluc) and is also referred to as the target/reporter construct. Cells were transfected with a plasmid encoding the functional nucleic acid under control of the U6 promoter and with the target/reporter (acceptor) construct. The donor is endogenous. **(B,C)** HEK293 cells were transfected with the target/reporter construct only (control) or co-transfected with the target/reporter and 50, 100 or 200 ng/well of a plasmid encoding the functional nucleic acid (KIF1B-TUBA1A) or a control (scramble-TUBA1A) and **(B)** luminescence was measured after 24 h. All samples were co-transfected with a plasmid encoding constitutively expressed renilla luciferase used for transfection normalization. **(C)** Using qPCR, fold change of *firefly luciferase* mRNA levels was analyzed with respect to a control sample transfected with the target/reporter only (control) and normalized to a house-keeping gene (GAPDH).

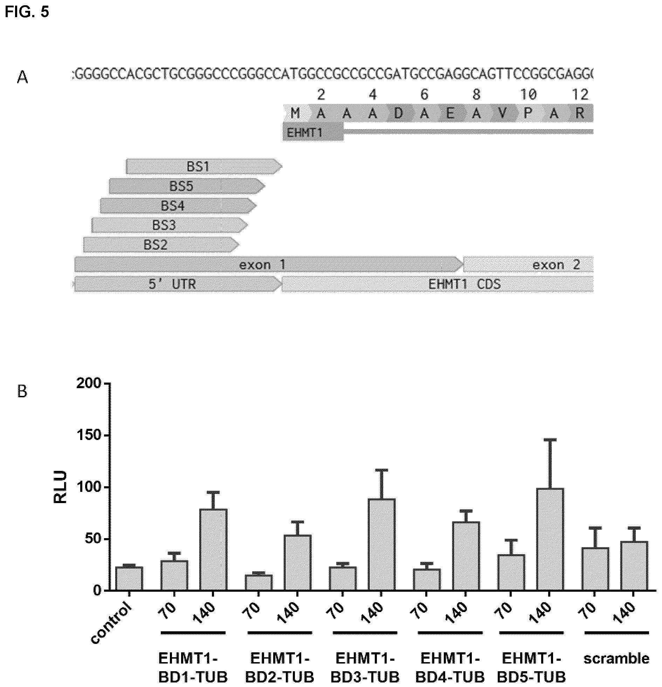

**FIG. 5****: (A)** Sequence of human EHMT 5'UTR and selected target (acceptor) binding sites (BD1-5) tested in the context of functional nucleic acid according to the invention. Each sequence complementary to the selected region of the 5'UTR of human EHMT1 (BD1-5) is followed by a 5nt linker followed by a sequence complementary to a region in the donor transcript, in this case TUBA1A 5'UTR. **(B)** HEK293 cells were transfected with the target/reporter construct only (control) or co-transfected with the target/reporter and 70 or 140 ng/well of a plasmid encoding the functional nucleic acid (EHMT1-BDX-TUB; X denoting BDs 1-5) or a control (scramble) and luminescence was measured after 24h. All samples were co-transfected with a plasmid encoding constitutively expressed renilla luciferase used for transfection normalization.

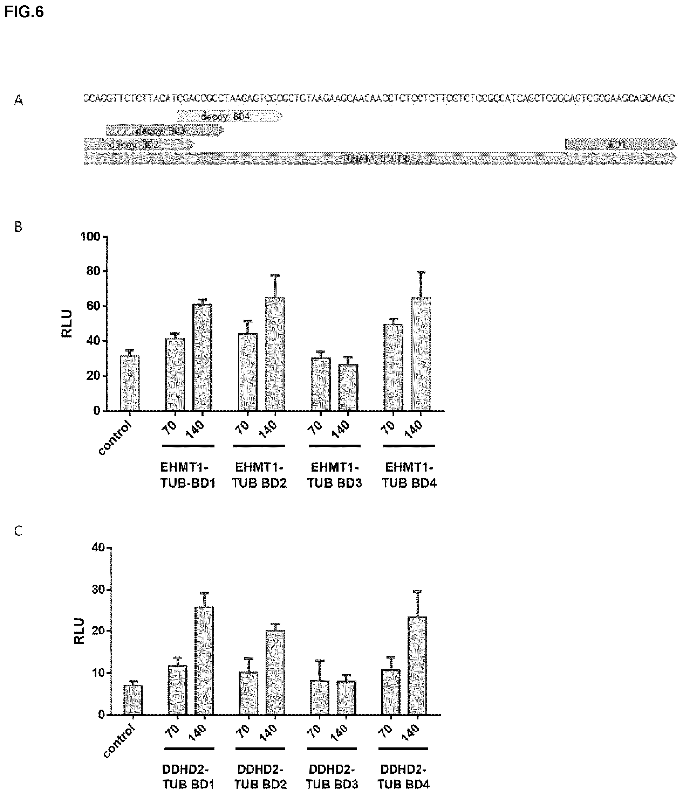

**FIG. 6****: (A)** Sequence of human TUBA1A 5'UTR and selected donor binding sites (BD1-4) tested in the context of functional nucleic acid according to the invention. Each sequence complementary to the selected region of the 5'UTR of human TUBA1A (BD1-4) is positioned in the functional nucleic acid downstream of a target (acceptor) binding sequence (in this case EHMT1 and DDHD2 5'UTR binding domains) and a 5nt linker sequence. **(B)** HEK293 cells were transfected with the target/reporter construct only (control; target/reporter construct is composed of an EHMT1 5'UTR sequence followed by a sequence encoding firefly luciferase) or co-transfected with the target/reporter and 70 or 140 ng/well of a plasmid encoding the functional nucleic acid (EHMT1- TUB-BDX; X denoting BDs 1-4) and luminescence was measured after 24h. (C) HEK293 cells were transfected with the target/reporter construct only (control; target/reporter construct is composed of an DDHD2 5'UTR sequence followed by a sequence encoding firefly luciferase) or co-transfected with the target/reporter and 70 or 140 ng/well of a plasmid encoding the functional nucleic acid (DDHD2-TUB-BDX; X denoting BDs 1-4) and luminescence was measured after 24h. All samples in (B) and (C) were co-transfected with a plasmid encoding constitutively expressed renilla luciferase used for transfection normalization.

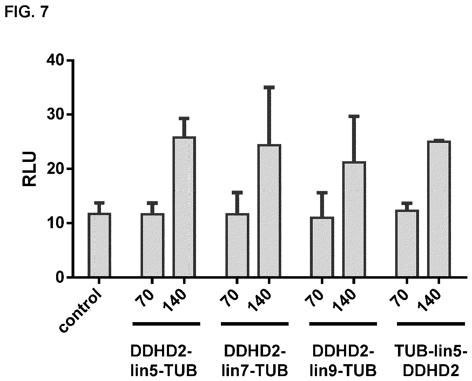

**FIG. 7****:** HEK293 cells were transfected with the target/reporter construct only (control; target/reporter construct is composed of an DDHD2 5'UTR sequence followed by a sequence encoding firefly luciferase) or co-transfected with the reporter and 70 or 140 ng/well of a plasmid encoding the functional nucleic acid, and luminescence was measured after 24 h. Functional nucleic acids are composed of sequences complementary to the target (acceptor) and donor transcripts connected with linkers of differing length (5, 7 or 9 nt; DDHD2-linX-TUB, wherein X depicts linker length). Additionally, an alternative 5' to 3' orientation of the functional nucleic acid was tested, wherein the donor complementary sequence is positioned at the 5' end, followed by a 5 nt linker and the target (acceptor) complementary sequence. All samples were co-transfected with a plasmid encoding constitutively expressed renilla luciferase used for transfection normalization.

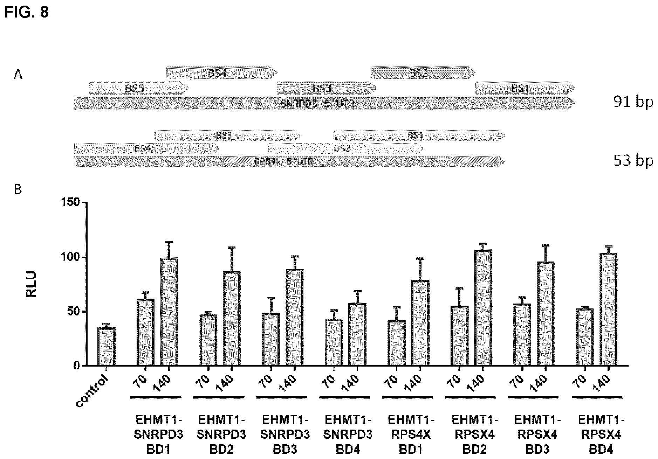

**FIG. 8****: (A)** Schematic of human SNRPD3 and RPS4X 5'UTRs and positions of selected donor binding sites (BDs1-4) tested in the context of functional nucleic acids according to the invention. Each sequence complementary to the selected region of the 5'UTR of human SNRPD3 or RPS4X (BD1-4) is positioned in the functional nucleic acid downstream of a target (acceptor) binding sequence (in this case EHMT1 5'UTR binding sequence) and a 5nt linker sequence. **(B)** HEK293 cells were transfected with the target/reporter construct only (control; target/reporter construct is composed of an EHMT1 5'UTR sequence followed by a sequence encoding firefly luciferase) or co-transfected with the target/reporter and 70 or 140 ng/well of a plasmid encoding the functional nucleic acid, and luminescence was measured after 24h. Functional nucleic acids are composed of sequences complementary to regions of the target (acceptor) and donor transcripts and are referred to as EHMT1-SNRPD3 BDX or EHMT1-RPS4X BDX wherein X depicts BDs from 1-4. All samples were co-transfected with a plasmid encoding constitutively expressed renilla luciferase used for transfection normalization.

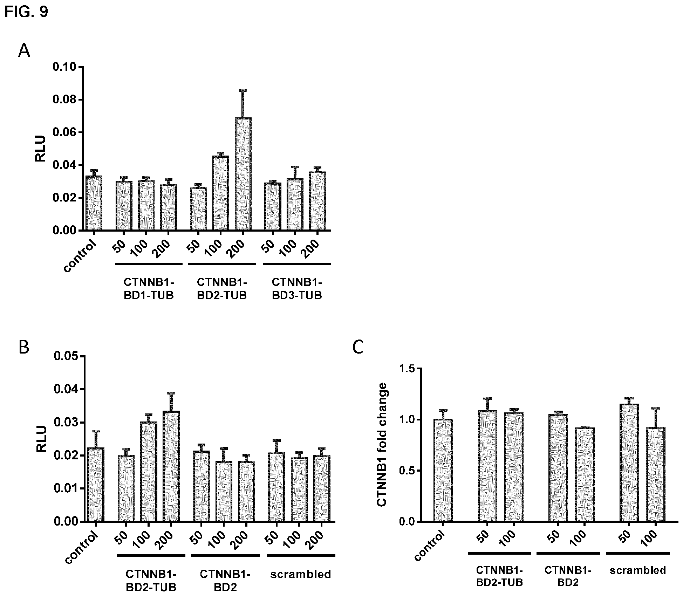

**FIG. 9****:** HEK293 cells were transfected with 50, 100 or 200nM functional nucleic acids in the form of chemically synthesized fully modified (PS backbone linkage and 2'-O-methoxyethyl (2'MOE) sugar modification) antisense oligonucleotides (ASOs). **(A, B)** The following day, cells were transfected with the TopFlash reporter plasmid, encoding a TCF/LEF binding site upstream of a sequence encoding firefly luciferase allowing for relative measurement of β-catenin levels. All samples were co-transfected with a plasmid encoding constitutively expressed renilla luciferase used for transfection normalization. Luminescence was measured 24 hours post reporter transfection. **(A)** ASOs were composed in the 5' to 3' orientation of a sequence complementary to the CTNNB1 5' (BD1 & 2) or 3' (BD3) UTR, followed by a 5 nt linker and a donor binding sequence, in this case complementary to the human TUBA1A 5'UTR. **(B)** Cells were transfected with an ASO composed of a sequence complementary to the 5' UTR of CTNNB1 followed by a 5 nt linker and a donor binding sequence, in this case complementary to the human TUBA1A 5' UTR (CTNNB1-BD2-TUB) or with control ASOs composed of only the CTNNB1 binding sequence (CTNNB1-BD2) or an ASO with a scrambled sequence (scrambled). **(C)** Using qPCR, fold change in *CTNNB1* mRNA levels was analyzed with respect to an untransfected control sample (control) and normalized to a house-keeping gene (GAPDH).

### Examples

The detailed examples given below serve to further illustrate and describe the invention without limiting its scope, providing a better understanding of the invention and its use.

### Summary of Experiments

The inventors selected as donors several housekeeping genes with respect to MRL prediction values, selecting candidates with the highest scores for testing as donor transcripts (table 1). Further, several candidate transcripts of genes involved in haploinsufficiencies were selected, including DDHD2, EHMT1 and CTNNB1. The predicted MRLs of the selected acceptor genes range from low to relatively high (table 1). For the initial testing of functional RNAs, they were cloned downstream of a U6 promotor for expression in cells. The reporter constructs were prepared harboring 5' UTR regions of target genes DDHD2, EHMT1, KIF1B, representing weak, moderate and strong UTRs, respectively, cloned upstream of an Fluc coding sequence. These genes are selected as causative of diseases that are known to be haploinsufficient and for which no therapy is currently available.

**Table 1. Predicion of mean ribosomal load (MRL) for selected housekeeping (HK) and haploinsufficient (HI) genes generated using the Optimus 5-Prime model**

| **HK gene** | **MRL** | **HI gene** | **MRL** |
|---|---|---|---|
| EEF1A1 | 6,47 | DDHD2 | 2,79 |
| C1ORF43 | 6,51 | NSD1 | 2,97 |
| GAPDH | 6,52 | GLI3 | 4,44 |
| HBB | 6,55 | EHMT1 | 5,01 |
| UBB | 6,61 | GATA2 | 5,07 |
| UBC | 6,71 | SYNGAP1 | 5,52 |
| UBA52 | 6,71 | ARSA | 5,75 |
| RPS4X | 6,78 | PMP22 | 6,18 |
| PGK1 | 6,79 | ABCD1 | 6,27 |
| SNRPD3 | 6,81 | CTNNB1 | 6,78 |
| TUBA1A | 6,89 | SATB2 | 6,92 |

The strength of UTRs for these genes was predicted using the Optimus 5-Prime model (table 1) (Sample et al., Nat. Biotechnol. 37, 803-809 (2019)). The functional oligonucleotide included an 18 nt stretch complementary to the 5' end of the acceptor transcript, in this case the reporter construct, followed by a 0 or 5 bp linker and an 18 nt sequence complementary to the donor transcript or in the opposite orientation. The donor transcripts which were evaluated include TUBA1A, SNRPD3 and RPSX4, all housekeeping genes with strong predicted 5' UTRs (table 1). Cells were co-transfected with functional oligonucleotide constructs and a reporter plasmid whereas the donor transcript was of endogenous origin, and luciferase intensity was measured. Short functional RNA molecules linking a highly translated donor mRNA transcript with a target mRNA through base complementarity resulted in a consistent increase of target protein from 1,5-3 fold when introduced into cells.

Further to demonstrate that this invention can facilitate the use of short nuclease resistant chemically synthesized oligonucleotides, which can be used in pharmacological preparations, chemically synthesized RNA molecules fully modified with PS bonds and 2'MOE ribose modifications were tested for upregulation of β-catenin. β-catenin acts as a transcriptional cofactor in the Wnt-signaling pathway and as an anchor in intracellular contacts and cell adhesion²⁰. CTNNB1 syndrome is caused by de novo occurrence of loss-of-function mutations within one allele of the CTNNB1 gene encoding β-catenin. The TopFlash assay allows measurement of a luciferase reporter under the control of a β-catenin responsive TCF/LEF promoter. Chemically modified oligonucleotides encompassing between 38 and 42 nucleotides were transfected into human cell lines together with the TopFlash reporter plasmid. The luminescence values reflecting β-catenin levels were measured resulting in 1,5-3-fold induction compared to a scrambled control oligonucleotide or in comparison to a control comprising only of the acceptor domain complementary to the target mRNA transcript without added donor domain.

### Example 1: Construction of reporter plasmids with UTR regions of weak, moderate and high predicted translation levels for evaluating the effect of functional nucleic acid on translation levels of target genes.

Relative mean ribosomal load (MRL) levels were predicted using the Optimus 5-Prime model (Sample et al., Nat. Biotechnol. 37, 803-809 (2019)) on a scale of 0-10 for several selected genes, mutations of which may result in a haploinsufficient condition. The genes selected were DDHD2, EHMT1 and KIF1B. DDHD2 encodes a phospholipase enzyme that participates in membrane trafficking between the ER and Golgi. Mutations can cause autosomal recessive spastic paraplegia 54. EHMT1 is a histone methyltransferase, involved in gene silencing. Loss of function deletions cause Kleefstra syndrome. KIF1B is a microtubule binding motor protein involved in chemical synaptic transmission and mutations are associated with neuroblastoma. These genes were selected to provide target transcripts / target mRNA according to the invention. Reporter plasmids were constructed for testing functional nucleic acids according to the invention. Reporters were constructed of a sequence encoding the 5'UTR of the human variant of each selected gene cloned upstream of a sequence encoding firefly luciferase. Sequences were cloned into the pCDNA3.1 expression vector for expression in mammalian cells. HEK293 cells were transfected with reporter plasmids and luminescence was measured 24h post transfection.

### Result:

As shown in FIG. 1, the expression level of each reporter plasmid correlated well with the predicted MRL level, indicating that these reporter constructs are appropriate for testing functional nucleic acids for increasing translation levels and will provide information on the feasibility of translation upregulation of genes with moderate to high endogenous translation.

### Example 2: Functional nucleic acid upregulates expression of a luciferase reporter with the 5'UTR region from human DDHD2 while not affecting luciferase mRNA levels.

Functional nucleic acids were designed to bridge two mRNA transcripts, the donor and target (acceptor) transcript according to the invention (FIG. 2A). The functional nucleic acid is composed of (from 5' to 3' orientation) a target (acceptor) binding sequence complementary to the 5'UTR of the target (acceptor) mRNA, in this case the 5'UTR of the human DDHD2 gene transcript. This motif is followed by a 5-nucleotide long linker and a sequence complementary to the donor 5' UTR, in this case the 5' UTR of the human TUBA1A mRNA. HEK293 cells were co-transfected with the reporter construct from example 1 and the functional nucleic acid or a scrambled control, both in the form of a plasmid under the control of a U6 promoter.

### Result:

Luminescence measurements correspond to the translation level of the reporter, i.e., the target mRNA. The activity is increased in a concentration-dependent manner when transfected with a functional nucleic acid targeting the 5'UTR of the target transcript, but not in cells transfected with a scrambled control (FIG. 2B). Conversely, the amount of mRNA of firefly luciferase, determined with qPCR, is not altered in cells, indicating that the effect of the functional nucleic acid indeed occurs on the translation, and not transcription level (FIG. 2C).

### Example 3: Functional nucleic acid upregulates expression of a luciferase reporter with the 5'UTR region from human EHMT1.

Functional nucleic acids were designed to connect two mRNA transcripts, the donor and target (acceptor) transcript according to the invention (FIG. 3A). The functional nucleic acid is composed of a target (acceptor) binding sequence complementary to the 5'UTR of the target mRNA (acceptor), in this case the 5'UTR of the human EHMT1 gene transcript. This motif is followed by a 5-nucleotide long linker and a sequence complementary to the donor 5' UTR, in this case the 5' UTR of the human TUBA1A gene transcript. HEK293 cells were co-transfected with the reporter construct from Example 1 and the functional nucleic acid or a scrambled control, both in the form of a plasmid under the control of a U6 promoter.

### Result:

Luminescence measurements indicate expression of the reporter construct. The levels are increased in a concentration dependent manner when transfected with a functional nucleic acid targeting the 5'UTR of the target transcript, but not in cells transfected with a scrambled control (FIG. 3B).

### Example 4: Functional nucleic acid upregulates expression of a luciferase reporter with the 5'UTR region from human KIF1B while not affecting luciferase mRNA levels.

Functional nucleic acids were designed to bridge two mRNA transcripts, the donor and target (acceptor) transcript according to the invention (FIG. 4A). The functional nucleic acid is composed of a target (acceptor) binding sequence complementary to the 5'UTR of the target mRNA (acceptor), in this case the 5'UTR of the human KIF1B gene transcript. This motif is followed by a 5-nucleotide long linker and a sequence complementary to the donor 5'UTR, in this case the 5'UTR of the human TUBA1A gene transcript. HEK cells were co-transfected with the reporter construct from Example 1 and the functional nucleic acid or a scrambled control, both in the form of a plasmid under the control of a U6 promoter.

### Result:

Luminescence measurements indicate expression of the reporter construct. The levels are increased in a concentration-dependent manner when transfected with a functional nucleic acid targeting the 5'UTR of the target transcript, but not in cells transfected with a scrambled control (FIG. 4B). Conversely, mRNA levels of firefly luciferase are not altered in cells, indicating that the effect of the functional nucleic acid is indeed on the translation, and not transcription level (FIG. 4C).

### Example 5: Functional nucleic acids targeting several sequences within the 5' UTR of the target (acceptor) transcript upregulate expression of a luciferase reporter with the 5'UTR region from human EHMT1.

Functional nucleic acids were designed containing target (acceptor) binding sequences complementary to different regions of the 5' UTR of the target (acceptor) transcript, in this case the 5' UTR of human EHMT1 transcript (FIG. 5A). The functional nucleic acid is composed of a target (acceptor) binding sequence complementary to the 5'UTR of the target mRNA (acceptor), in this case an 18bp long sequence within the 5'UTR of the human EHMT1 gene transcript. This motif is followed by a 5-nucleotide long linker and a sequence complementary to the donor 5'UTR, in this case the 5'UTR of the human TUBA1A gene transcript. HEK293 cells were co-transfected with the reporter construct from Example 1 and the indicated functional nucleic acids or a scrambled control, both in the form of a plasmid under the control of a U6 promoter (FIG. 5B).

### Result:

Luminescence measurements correspond to the translation level of the reporter. The levels are increased in a concentration-dependent manner when transfected with a functional nucleic acid targeting the 5'UTR of the target transcript, but not in cells transfected with a scrambled control (FIG. 5B). Effector RNAs (functional nucleic acids) targeting different areas of the EHMT1 5' UTR increase luciferase expression to a similar extent.

### Example 6: Functional nucleic acids targeting several sequences within the 5' UTR of the donor transcript upregulate expression of luciferase reporters with the 5'UTR region from human EHMT1 or DDHD2.

Functional nucleic acids were designed containing donor binding sequences complementary to different regions of the 5' UTR of the donor transcript, in this case the 5' UTR of human TUBA1A (FIG. 6A). The functional nucleic acid is composed of a target (acceptor) binding sequence complementary to the 5'UTR of the target mRNA (acceptor), in this case an 18bp long sequence within the 5'UTR of the human EHMT1 transcript (FIG. 6B) or DDHD2 gene transcript (FIG. 6C). This motif is followed by a 5-nucleotide long linker and a sequence complementary to a region in the donor 5' UTR, in this case a depicted region within the 5' UTR of the human TUBA1A gene. HEK cells were co-transfected with a reporter construct from Example 1 (5' UTR EHMT1-Fluc (FIG. 6B) or 5' UTR EHMT1-Fluc (FIG. 6C)) and the indicated functional nucleic acids or a scrambled control, both in the form of a plasmid under the control of a U6 promoter (FIG. 6B,C)).

### Result:

Luminescence measurements indicate expression of the reporter construct. The levels are increased in a concentration dependent manner when transfected with 3 out of 4 tested functional nucleic acid variants, differing in the binding site of the donor binding sequence. All the donor binding sequences target specific sequences within the 5' UTR of human TUBA1A, as depicted in FIG. 6A.

### Example 7: Linker length does not affect function of the effector RNA

The functional nucleic acid is composed of a target (acceptor) binding sequence complementary to the 5'UTR of the target mRNA (acceptor), in this case an 18bp long sequence within the 5'UTR of the human DDHD2 mRNA. This motif is followed by a linker of different length and a sequence complementary to the donor 5' UTR, in this case a depicted region within the 5' UTR of the human TUBA1A gene transcript. Different linker lengths were tested (5, 7 and 9 nt). HEK293 cells were co-transfected with a reporter construct from Example 1 (5' UTR DDHD2-Fluc) and indicated functional nucleic acids in the form of a plasmid under the control of a U6 promoter (FIG. 7). Additionally, a functional nucleic acid with the opposite 5' to 3' orientation was tested, i.e., where the donor binding sequence is upstream (5') of the linker and target binding sequence.

### Result:

Luminescence measurements indicate expression of the reporter construct. The levels are increased in a concentration dependent manner when transfected with functional nucleic acids with different linker lengths, leading to the conclusion that a linker between 5 and 9 nt works equally well in the context of the functional nucleic acids according to the invention. Furthermore, relative position of the donor and acceptor binding domains with respect to each other do not affect the result, as seen in FIG. 7.

### Example 8: Housekeeping genes SNRPD3 and RPSX4 also function as functional donor transcripts

Additional mRNA candidate transcripts were tested as feasible donor transcripts according to the invention. The functional nucleic acid is composed of a target (acceptor) binding sequence complementary to the 5'UTR of the target mRNA (acceptor), in this case an 18 bp long sequence within the 5'UTR of the human EHMT1 transcript. This motif is followed by a 5-nucleotide long linker and a sequence complementary to the donor 5' UTR, in this case the 5' UTR region of the transcripts of genes SNRPD3 and RPSX4. Both genes are ubiquitously expressed in all cells and tissues and have high predicted levels of translation. HEK293 cells were co-transfected with a reporter construct from example 1 (5' UTR EHMT1-Fluc) and the indicated functional nucleic acids in the form of a plasmid under the control of a U6 promoter (FIG. 8).

### Result:

Luminescence measurements indicate expression of the reporter construct. The levels are increased in a concentration dependent manner when transfected with functional nucleic acids targeting different regions within the 5' UTRs of both tested donors, demonstrating that different housekeeping genes may be used as donor transcripts according to the invention.

### Example 9: The functional nucleic acid delivered in the form of a chemically synthesized, fully PS 2'MOE modified oligonucleotide is fully functional for upregulation of protein translation

Modified antisense oligonucleotides provide an attractive option in therapeutic settings due to a high safety profile and a good pharmacodynamic, pharmacokinetic, and biodistribution profile. Useful modifications include phosphorothioate (PS) internucleotide bonds and a 2'-O-methoxyethyl (2'MOE) modification on the ribose sugar of ribonucleic acids. Here, the delivery of functional nucleic acids in the form of synthetic ASOs was shown to be functional for upregulation of target protein levels. Functional nucleic acid in the form of fully modified PS 2'MOE ASOs targeting the 5' or 3' UTR of CTNNB1 and TUBA1A were delivered to HEK293 cells. The functional nucleic acid is composed of an target (acceptor) binding domain complementary to the 5'UTR of the target mRNA (acceptor), in this case an 18bp long sequence within the 5'UTR (BD1 and 2) or the 3' UTR (BD3) of the human CTNNB1 gene transcript. This motif is followed by a 5-nucleotide long linker and a sequence complementary to the donor 5' UTR, in this case the 5' UTR region of human TUBA1A mRNA. Cells were then transfected with a TopFlash reporter plasmid constructed or LEF/TCF binding sites upstream of a sequence encoding firefly luciferase. The reporter plasmid expression is used for measuring relative β-catenin protein activity.

### Result:

Luminescence measurements correlate to the expression of the reporter construct, wherein a functional nucleic acid variant (CTNNB1-BD2-TUB) targeting a region within the 5' UTR of CTNNB1 increased reporter levels in a concentration dependent manner (FIG. 9A). Reporter expression was not enhanced in cells transfected with an ASO containing only the acceptor binding domain (CTNNB1-BD2) or an ASO with a scrambled sequence (FIG. 9B). In contrast, mRNA levels were not increased in cells transfected with the indicated ASOs, demonstrating that the effect is due to enhanced translation rather than enhanced transcription or mRNA stabilization.

### Sequence list:

SEQ ID NO: 1 (description):
   (sequence as text, such as gate..)
SEQ ID NO: 1 target binding sequence reverse complementary to 5'UTR of human DDHD2 (Fig 2,6,7)
   TTCGCTCTCTGGCGGCCC
SEQ ID NO: 2 donor binding sequence reverse complementary to 5'UTR of human TUBA1A (Fig 2-7,9)
   GGTTGCTGCTTCGCGACTG
SEQ ID NO: 3 target binding sequence reverse complementary to 5'UTR of human EHMT1 (Fig3,5,6,8)
   GGCCCGGGCCCGCAGCGT
SEQ ID NO: 4 target binding sequence reverse complementary to 5'UTR of human KIF1B (Fig 4)
   TTTAATGGCCTTGTTATA
SEQ ID NO: 5 donor binding sequence reverse complementary to 5'UTR of human TUBA1A (TUB-BD1) (Fig 6)
   GGTTGCTGCTTCGCGACTG
SEQ ID NO: 6 donor binding sequence reverse complementary to 5'UTR of human TUBA1A (TUB-BD2) (Fig 6)
   TCGATGTAAGAGAACCTGC
SEQ ID NO: 7 donor binding sequence reverse complementary to 5'UTR of human TUBA1A (TUB-BD3) (Fig 6)
   GGCGGTCGATGTAAGAGAAC
SEQ ID NO: 8 donor binding sequence reverse complementary to 5'UTR of human TUBA1A (TUB-BD4) (Fig 6)
   GCGACTCTTAGGCGGTCG
SEQ ID NO: 9 sequence of 5nt long linker (Fig 7)
   ttatt
SEQ ID NO: 10 sequence of 7nt long linker (Fig 7)
   ttatttt
SEQ ID NO: 11 sequence of 9nt long linker (Fig 7)
   ttattttct
SEQ ID NO: 12 donor binding sequence reverse complementary to 5'UTR of human SNRPD3 (SNRPD3 BD1) (Fig 8)
   CTTGGCAGGAAGAGAGTT
SEQ ID NO: 13 donor binding sequence reverse complementary to 5'UTR of human SNRPD3 (SNRPD3 BD2) (Fig 8)
   CGGCTCTGGCCTACTTTTC
SEQ ID NO: 14 donor binding sequence reverse complementary to 5'UTR of human SNRPD3 (SNRPD3 BD3) (Fig 8)
   CTTCTCTTCGGTCCGCTG
SEQ ID NO: 15 donor binding sequence reverse complementary to 5'UTR of human SNRPD3 (SNRPD3 BD4) (Fig 8)
   CGAAAGATGCTACGGCGAAG
SEQ ID NO: 16 donor binding sequence reverse complementary to 5'UTR of human RPSX4 (RPSX4 BD1) (Fig 8)
   GGCTGCGTTAGGCAAGGAAAG
SEQ ID NO: 17 donor binding sequence reverse complementary to 5'UTR of human RPSX4 (RPSX4 BD2) (Fig 8)
   GGCAAGGAAAGAGGACCTC
SEQ ID NO: 18 donor binding sequence reverse complementary to 5'UTR of human RPSX4 (RPSX4 BD3) (Fig 8)
   CCTCCGTCTTCCGGTGCG
SEQ ID NO: 19 donor binding sequence reverse complementary to 5'UTR of human RPSX4 (RPSX4 BD4) (Fig 8)
   CCGGTGCGCGTAGAAATT
SEQ ID NO: 20 target binding sequence reverse complementary to 5'UTR of human CTNNB1 (CTNNB1 BD1) (Fig 8)
   GACCGTCCTCGACCTGCG
SEQ ID NO: 21 target binding sequence reverse complementary to 5'UTR of human CTNNB1 (CTNNB1 BD2) (Fig 8)
   TATACTTCAAATACCCTC
SEQ ID NO: 22 target binding sequence reverse complementary to 5'UTR of human CTNNB1 (CTNNB1 BD3) (Fig 8)
   CAGGGGAACAGGCTCCTC

The present invention is summarized by the following items:

### [Nucleic acid]

1. A functional nucleic acid comprising at least one of each of the two domains (A) and (B), preferably comprising (A) and (B):
   (A) being a target binding sequence essentially reverse complementary to a region of a target mRNA sequence for which protein translation in a cell is to be enhanced, and
   (B) being a donor binding sequence essentially reverse complementary to a region of a donor mRNA sequence which has a high translational activity in said cell.
2. The functional nucleic acid according to item 1, which is an RNA molecule, preferably a nonmodified RNA molecule or a chemically modified RNA molecule.
3. The functional nucleic acid according to any one of the preceding items, which is a single-stranded nucleic acid molecule.
4. The functional nucleic acid according to any one of the preceding items, which does not form stable intramolecular secondary structures under physiological conditions.
5. The functional nucleic acid according to any one of the preceding items, which is an isolated nucleic acid molecule, preferably produced by chemical synthesis.
6. The functional nucleic acid according to any one of the preceding items, comprising at least one chemical modification compared to a nonmodified nucleic acid.
7. The functional nucleic acid according to item 6, wherein at least one internucleosidic linkage in the nucleic acid molecule is a modified internucleosidic linkage, preferably a modified nuclease resistant internucleosidic linkage, more preferably a phosphorothioate linkage.
8. The functional nucleic acid according to item 7, wherein at least two internucleosidic linkages in the functional nucleic acid are modified internucleosidic linkages, preferably wherein at least 20% of the internucleosidic linkages are modified internucleosidic linkages.
9. The functional nucleic acid according to any one of items 6-8, wherein at least one nucleotide is a 2'-modified ribonucleotide, preferably selected from the group consisting of 2'-O-methylribonucleotide, 2'fluororibonucleotide and 2'-O-(2-methoxyethyl)-ribonucleotide.
10. The functional nucleic acid according to any one of items 6-9, wherein at least one nucleotide is a 2'-4'-bridged nucleotide, preferably a locked nucleotide having a 2'-O-4'-C-methylene bridge.
11. The functional nucleic acid according to any one of the preceding items, wherein functional nucleic acid has a total length of 20-100 nucleotides, preferably 18-50 nucleotides, more preferably 25-50 nucleotides.
12. The functional nucleic acid according to any one of the preceding items, wherein the functional nucleic acid is covalently or non-covalently linked to another biopolymer, preferably to another nucleic acid or a protein.

### [Target binding sequence]

13. The functional nucleic acid according to any one of the preceding items, wherein the target binding sequence has a length of 8-50 nucleotides, preferably of 14-22 nucleotides.
14. The functional nucleic acid according to any one of the preceding items, wherein the target binding sequence is essentially reverse complementary to a region of the target mRNA sequence which does not form and is not part of a stable intramolecular secondary structure under physiological conditions.
15. The functional nucleic acid according to any one of the preceding items, wherein the target binding sequence is essentially reverse complementary to a region comprised in the 5' untranslated region (5'UTR), the coding sequence (CDS) and/or the 3' untranslated region (3'UTR) of the target mRNA.
16. The functional nucleic acid according to any one of the preceding items, wherein the target binding sequence is essentially reverse complementary to a region comprised in the 5' untranslated region (5'UTR) of the target mRNA.
17. The functional nucleic acid according to any one of the preceding items, wherein the target binding sequence is at least 80% complementary to a region of the target mRNA sequence.
18. The functional nucleic acid according to any one of the preceding items, wherein the target binding sequence comprises one or more segments fully complementary to a region of the target mRNA sequence, wherein each segment is at least 6 nucleotides long.
19. The functional nucleic acid according to any one of the preceding items, wherein the target binding sequence is fully reverse complementary to a region of the target mRNA sequence.

### [Donor binding sequence]

20. The functional nucleic acid according to any one of the preceding items, wherein the donor binding sequence has a length of 6-50 nucleotides, preferably of 6-22 nucleotides.
21. The functional nucleic acid according to any one of the preceding items, wherein the donor-binding sequence is essentially reverse complementary to a region of the donor mRNA sequence which does not form and is not part of a stable intramolecular secondary structure under physiological conditions.
22. The functional nucleic acid according to any one of the preceding items, wherein the donor binding sequence is reverse complementary to a region comprised in the 5' untranslated region (5'UTR), the coding sequence (CDS) and/or the 3' untranslated region (3'UTR) of the donor mRNA.
23. The functional nucleic acid according to item 22, wherein the donor binding sequence is reverse complementary to a region comprised in the 5' untranslated region (5'UTR) of the donor mRNA.
24. The functional nucleic acid according to any one of the preceding items, wherein the donor binding sequence is at least 80% complementary to a region of the donor mRNA sequence.
25. The functional nucleic acid according to any one of the preceding items, wherein the donor binding sequence comprises one or more segments fully complementary to a region of the donor mRNA sequence, wherein each segment is at least 6 nucleotides long.
26. The functional nucleic acid according to any one of the preceding items, wherein the donor binding sequence is fully reverse complementary to a region of the donor mRNA sequence.
27. The functional nucleic acid according to any one of the preceding items, wherein the donor mRNA is a transcript of a constitutively expressed gene, also called a housekeeping gene.
28. The functional nucleic acid according to any one of the preceding items, wherein the donor mRNA is a transcript of a gene which is highly expressed within a specific cell type or tissue or at specific cell state.
29. The functional nucleic acid according to any one of items 1-28, wherein the donor mRNA is a transcript of a gene which is highly expressed under certain physiological conditions or in a specific cell state.
30. The functional nucleic acid according to any one of the preceding items, wherein the donor mRNA is more abundant in the cell than the target mRNA.
31. The functional nucleic acid according to any one of the preceding items, wherein the donor mRNA and the target mRNA are present in the cell in a ratio of 1.5:1 to 10:1.
32. The functional nucleic acid according to any one of the preceding items, wherein the ratio of predicted mean ribosome load (MRL) of the donor mRNA to MRL of the target mRNA is 1.2:1 to 3:1.
33. The functional nucleic acid according to any one of the preceding items, wherein the donor mRNA encodes a protein selected from the group consisting of TUBA1A, SNRPD3, RPSX4, RPSX4, EEF1G, ACTB, HPRT, TPT1, YWHAZ, VPS29, RPLPO, RPLPO, B2M, SDHA, PSMB2, CHMP2A, ACTG1, RPL13A, RPL12, EEF1A1, C1orf43, GAPDH, HBB, UBB, UBC, UBA52 and PGK1.
34. The functional nucleic acid according to any one of the preceding items, wherein the donor binding sequence is a sequence with at least 75% identity, preferably at least 90% identity to a sequence selected from the group consisting of SEQ ID NO: 2, 5-8 and 12-19 or comprises a subsequence thereof comprising at least 8 consecutive nucleotides.
35. The functional nucleic acid according to any one of the preceding items, wherein the donor binding sequence is a sequence selected from the group consisting of SEQ ID NO: 2, 5-8 and 12-19.

### [Spacer/linker]

36. The functional nucleic acid according to any one of the preceding items, further comprising a linker sequence between the target binding sequence and the donor binding sequence.
37. The functional nucleic acid according to item 36, wherein the linker sequence is not complementary to the target mRNA or the donor mRNA.
38. The functional nucleic acid according to item 36 or 37, wherein the linker sequence has a length of 1-50 nucleotides, preferably of 2-10 nucleotides.

### [DNA / expression vector]

39. DNA encoding the functional nucleic acid according to any one of the preceding items.
40. An expression vector comprising a sequence encoding the functional nucleic acid according to any one of items 1-38.
41. The expression vector according to item 40, wherein the expression vector is an expression vector for transient or stable production of the functional nucleic acid according to any one of items 1-38 in the target cell, preferably wherein the expression vector is selected from a plasmid, linear or branched DNA or a viral vector, wherein the viral vector is preferably selected from a lentiviral, retroviral, adenoviral or AAV vector.

### [Composition]

42. A composition comprising the functional nucleic acid according to any one of items 1-38, the DNA according to item 39 or the expression vector according to item 40 or 41.

### [Method]

43. An in vitro method of enhancing translation of a target mRNA sequence in an isolated cell, comprising the steps of:
   (i) providing a functional nucleic acid according to any of items 1-38, a DNA according to item 39 or an expression vector according to item 40 or 41, and
   (ii) delivering the functional nucleic acid, DNA or expression vector to the cell.
44. The in vitro method of item 43, wherein the functional nucleic acid is provided in step (i) by chemical synthesis.
45. The in vitro method of any one of items 43 or 44, wherein the isolated cell is a eukaryotic or prokaryotic cell, preferably a mammalian cell, more preferably an isolated human cell.

### [Use]

46. Use of a functional nucleic acid according to any one of items 1-38, a DNA according to item 39 or an expression vector according to item 40 or 41 for enhancing protein translation from a target mRNA sequence.
47. Use of a functional nucleic acid, a DNA or an expression vector according to item 46 for research and/or recombinant protein production.

### [Nucleic acid for use in a method of treatment]

48. A functional nucleic acid according to any one of items 1-38 for use in a method of treating a disease or condition caused by reduced gene dosage or reduced gene expression.
49. The functional nucleic acid for use according to item 48 for use in a method of treating a disease or condition caused by haploinsufficiency of a gene.
50. A composition comprising the functional nucleic acid according to any one of items 1-38 for use in a method of treating or preventing a disease or condition caused by reduced gene dosage or reduced gene expression.
51. A composition comprising the functional nucleic acid according to any one of items 1-38 for use in a method of treating or preventing a disease or condition by the increased production of a selected protein or a group of proteins.

## Claims

1. A functional nucleic acid comprising at least one of each of the two domains (A) and (B),
(A) being a target binding sequence essentially reverse complementary to a region of a target mRNA sequence for which protein translation in a cell is to be enhanced, and
(B) being a donor binding sequence essentially reverse complementary to a region of a donor mRNA sequence which has a higher translational activity than the target mRNA sequence in said cell.

2. The functional nucleic molecule according to claim 1, which is an RNA molecule, preferably a nonmodified RNA molecule or a chemically modified RNA molecule comprising at least one chemical modification compared to a nonmodified RNA molecule.

3. The functional nucleic acid according to any one of the preceding claims, which is a single-stranded nucleic acid molecule, which preferably does not form stable intramolecular secondary structures under physiological conditions.

4. The functional nucleic acid according to any one of the preceding claims,
wherein the donor binding sequence is located 5' or 3' of the target binding sequence and/or
wherein the functional nucleic acid further comprises a linker sequence between the target binding sequence and the donor binding sequence, preferably a linker comprising 1-10 nucleotides.

5. The functional nucleic acid according to any one of the preceding claims,
wherein the target binding sequence is fully reverse complementary to a region of the target mRNA sequence and/or
wherein the donor binding sequence is fully reverse complementary to a region of the donor mRNA sequence.

6. The functional nucleic acid according to any one of the preceding claims, wherein
- the target binding sequence has a length of 6-50 nucleotides, preferably of 14-22 nucleotides, and/or
- the donor binding sequence has a length of 6-50 nucleotides, preferably of 6-22 nucleotides, and/or
- the functional nucleic acid has a total length of 20-100 nucleotides.

7. The functional nucleic acid according to any one of the preceding claims, wherein
- the target binding sequence is essentially reverse complementary to a region comprised in the 5' untranslated region (5'UTR), the coding sequence (CDS) and/or the 3' untranslated region (3'UTR) of the target mRNA, preferably in the 5'UTR of the target mRNA, and/or
- the donor binding sequence is reverse complementary to a region comprised in the 5' untranslated region (5'UTR), the coding sequence (CDS) and/or the 3' untranslated region (3'UTR) of the donor mRNA, preferably in the 5'UTR of the donor mRNA.

8. The functional nucleic acid according to any one of the preceding claims, wherein the donor mRNA is more abundant in the cell than the target mRNA,
preferably wherein donor mRNA and the target mRNA are present in the cell in a ratio of 1.5:1 to 10:1, and/or
wherein the ratio of predicted mean ribosome load (MRL) of the donor mRNA to MRL of the target mRNA is 1.2:1 to 3:1,
preferably wherein the donor mRNA is a transcript of a constitutively expressed gene, also called a housekeeping gene,
more preferably wherein the donor mRNA encodes a protein selected from the group consisting of TUBA1A, SNRPD3, RPSX4, RPSX4, EEF1G, ACTB, HPRT, TPT1, YWHAZ, VPS29, RPLPO, RPLPO, B2M, SDHA, PSMB2, CHMP2A, ACTG1, RPL13A, RPL12, EEF1A1, C1orf43, GAPDH, HBB, UBB, UBC, UBA52 and PGK1.

9. The functional nucleic acid according to any one of the preceding claims, wherein the donor binding sequence is a sequence having at least 75% homology, preferably a sequence having at least 90% homology, more preferably a sequence identical to a sequence selected from the group consisting of SEQ ID NO: 2, 5-8, 12-19.

10. DNA encoding the functional nucleic acid according to any of the preceding claims.

11. An expression vector comprising a sequence encoding the functional nucleic acid according to any one of claims 1-9.

12. A composition comprising the functional nucleic acid according to any one of claims 1-9, the DNA according to claim 10 or the expression vector according to claim 11.

13. An in vitro method of enhancing translation of a target mRNA sequence in an isolated cell, comprising the steps of:
(i) providing a functional nucleic acid according to any of claims 1-9, a DNA according to claim 10 or an expression vector according to claim 11, and
(ii) delivering the functional nucleic acid, DNA or expression vector to the cell,
wherein the isolated cell is preferably a eukaryotic cell, more preferably a mammalian cell, particularly an isolated human cell.

14. Use of a functional nucleic acid according to any of claims 1-9, a DNA according to claim 10 or an expression vector according to claim 11 for enhancing protein translation from a target mRNA sequence.

15. A functional nucleic acid according to any one of claims 1-9 for use in a method of treating a disease or condition caused by reduced gene dosage, such as by haploinsufficiency of a gene, or by reduced gene expression.

16. A functional nucleic acid according to any one of claims 1-9 for use in a method of treating a disease or condition by increased production of a protein or a group of proteins.
